(19) Europäisches Patentamt
European Patent Office
Office européen des brevets

(11) Publication number: **0 146 067 A2**

(12) **EUROPEAN PATENT APPLICATION**

(21) Application number: **84114651.7**

(22) Date of filing: **03.12.84**

(51) Int. Cl.⁴: **C 07 C 101/02**, C 07 C 101/72, C 07 C 149/243, C 07 D 209/20, C 07 D 209/34, C 07 D 311/76, C 07 D 217/02, C 07 K 5/06, A 61 K 31/195, A 61 K 31/35, A 61 K 31/395

(30) Priority: **06.12.83 US 558643**
**09.11.84 US 669342**

(43) Date of publication of application: **26.06.85**
**Bulletin 85/26**

(84) Designated Contracting States: **AT BE CH DE FR GB IT LI LU NL SE**

(71) Applicant: **MERRELL DOW PHARMACEUTICALS INC.,**
**2110 E. Galbraith Road, Cincinnati Ohio 45215 (US)**

(72) Inventor: **Seiler, Nikolaus, 35, Rue de la Redoute,**
**F-67100 Strasbourg (FR)**

(74) Representative: **Sgarbi, Renato et al, Gruppo Lepetit S.p.A. Patent and Trademark Dept. Via Roberto Lepetit, 8, I-20124 Milano (IT)**

(54) **Novel substrates for D-amino acid oxidase.**

(57) This invention relates to substrates of D-amino acid oxidase which are valuable therapeutic agents. More particularly, the compounds of the present invention includes a D-amino acid oxidase substrate of the structural formula

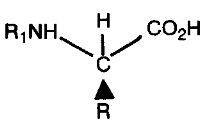

and the pharmaceutically acceptable salts thereof, wherein R is H, $-CH_3$, $-CH_3CH_2-$, $CH_3CH_2CH_2-$, $CH_3(CH_2)_2CH_2-$, $(CH_3)_2CH-$,

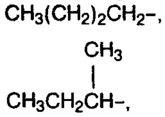

$-CH_2OH$, $CH_3CH_2SCH_2CH_2-$,

$CH_3SCH_2-$, $CH_3SCH_2CH_2-$,

$C_6H_5CH_2-$, 4-OH-$C_6H_4CH_2-$,

3,4-diOH-$C_6H_3CH_2-$ and

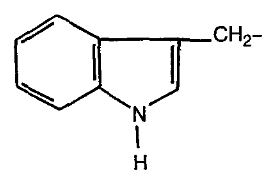

and $R_1$ is the des-amino residue of a $-NH_2$ bearing therapeutic agent.

EP 0 146 067 A2

# NOVEL SUBSTRATES FOR D-AMINO ACID OXIDASE

This invention relates to novel amino acid derivatives, to the methods of their preparation and to their use as novel therapeutic agents.

More particularly, this invention relates to D-amino acid derivatives which are substrates of the enzyme D-amino acid oxidase, to the methods for their preparation and to their use as therapeutic agents.

Still more particularly, this invention relates to D-amino acid derivatives which are substrates for the enzyme D-amino acid oxidase, said derivatives having the formula:

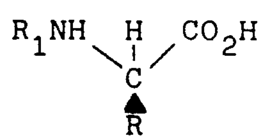

(I)

wherein:

R is H, $CH_3$, $CH_3CH_2$, $CH_3CH_2CH_2$, $CH_3(CH_2)_2CH_2$, $(CH_3)_2CH$, $CH_3CH_2\overset{CH_3}{\overset{|}{C}}H$, $HOCH_2$, $CH_3CH_2SCH_2CH_2$, $CH_3SCH_2$, $CH_3SCH_2CH_2$, $C_6H_5CH_2$, 4-hydroxy-$C_6H_4CH_2$, 3,4-dihydroxy-$C_6H_3CH_2$ and

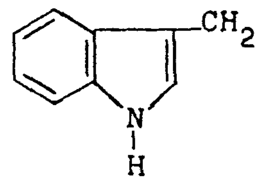
;

$R_1$ represents the des-amino residue of a therapeutic agent bearing an active primary amine; and the pharmaceutically acceptable salts thereof.

It should be emphasized that with but one exception the amino acid moiety represented in formula (I) above, must in each instance be in the D-enantiomorphic form, as depicted by the heavy bond attached to the symbol R. The sole exception is the amino acid glycine which, of course, does not possess an assymetric center.

It should be further noted that in order to be a suitable substrate for the enzyme D-amino acid oxidase, the α-amino nitrogen must be in the form of a secondary amine in order to create an active primary amine of the therapeutic agent being generated. It cannot represent a tertiary amine. This can be schematically illustrated as follows:

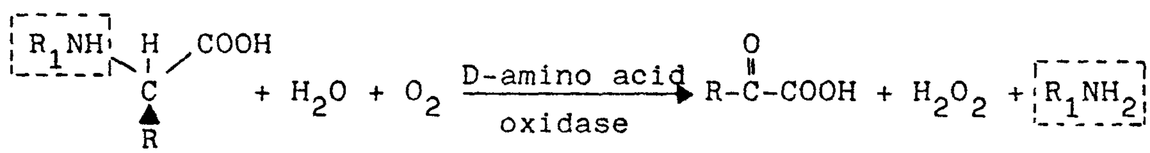


(I)

In essence, this invention relates to compounds comprising (a) a primary amino therapeutic moiety or reactive derivative thereof, and (b) a D-amino acid moiety having the formula

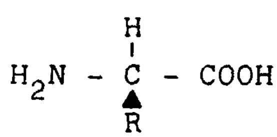

or reactive derivative thereof, which compounds are capable of *in vivo* enzymatic cleavage when in contact with the enzyme D-amino acid oxidase. Thus, such cleavage at the site of D-amino acid oxidase will generate the primary amine bearing therapeutic agent which will, in most instances, more effectively be available to treat the disease condition(s) for which the therapeutic agent is known to be useful. For example, the compound N-[(E)-3-fluoro-2-(3-trifluoromethylphenyl)-2-propenyl]glycine, prepared via the reactive derivatives of glycine and the antidepressant (E)-2-(3-trifluoromethylphenyl)-3-fluoroallylamine will exert an antidepressant effect in mammals by virtue of the fact that it is a substrate for D-amino acid oxidase.

Thus, this invention quite naturally embraces the D-amino acid substrates of many diverse compounds selected from a wide variety of therapeutic agents. In general, the compounds of formula (I) have an enhanced, but similar therapeutic activity to that which is known for the amino bearing therapeutic agents _per se_.

Two classes of primary amino bearing therapeutic agents, however, do not serve as substrates for D-amino acid oxidase. First, therapeutic agents having an aromatic ring or an aromatic heterocycle adjacent to the primary amine do not function as D-amino acid substrates. Secondly, therapeutic agents having a carboxyl or carbalkoxy group α to the primary amine do not serve as D-amino acid substrates.

Examples of primary amino bearing therapeutic agents which do not form D-amino acid substrates include:

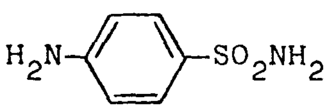

Sulfanilamide

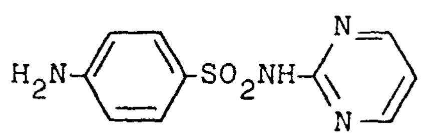

Sulfadiazine

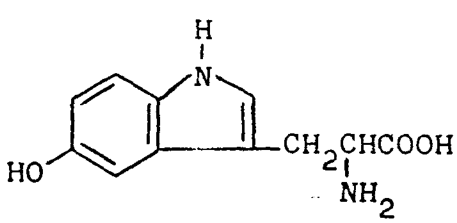

5-Hydroxytryptophan

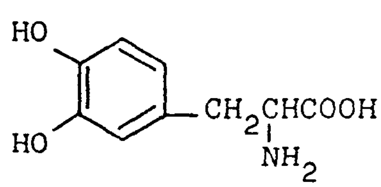

Dopa

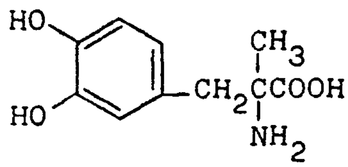

Methyldopa

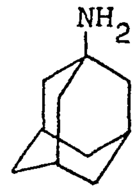

Amantadine

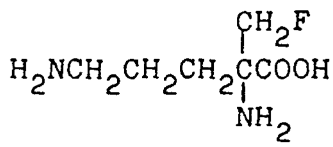

α-Monofluoromethylornithine

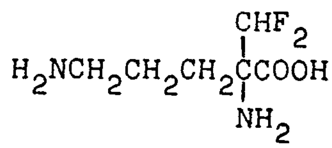

α-Difluoromethylornithine

In general, the preferred D-amino acid derivatives of formula (I) are those wherein R represents a residue of glycine (R is H), alanine (R is $-CH_3$), valine [R is $(CH_3)_2CH-$], methionine (R is $CH_3SCH_2CH_2-$), and methyl cysteine (R is $CH_3SCH_2-$), with the residues of glycine, alanine and valine being the most preferred.

The $NH_2$ bearing $R_1$ therapeutic agents can be antihypertensives, vasodilating dopamine agonists, anorectic agents, anti-migraine agents, anti-depressants, antibacterial, antifungal, antiviral and antiprotozoal agents, diuretics, anti-epileptics, cytotoxic agents, anti-anxiety agents, centrally acting antihypertensive agents, Angiotensin Converting Enzyme (ACE) inhibitors useful as antihypertensive agents and the like.

The preferred antihypertensive agents of formula (I) are the D-amino acid oxidase substrates having the formula:

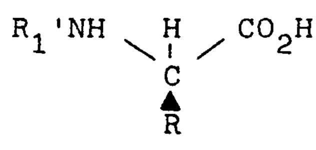

(Ia)

wherein the R moiety is as defined for formula I and $R_1'$ is represented by

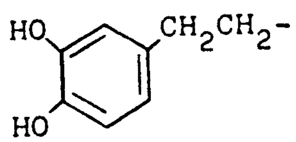

,

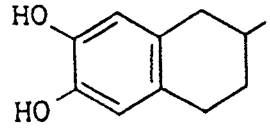

,

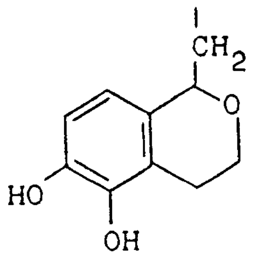

,

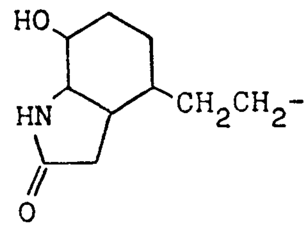

,

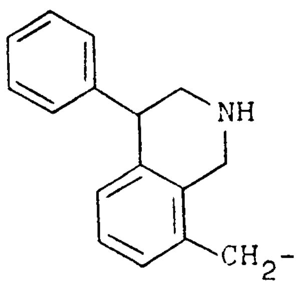

,

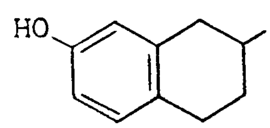

,

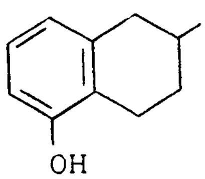

,

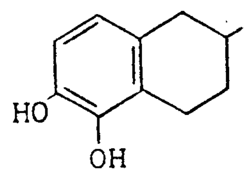

,

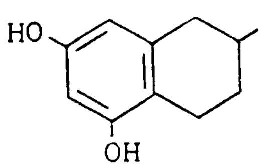

, and

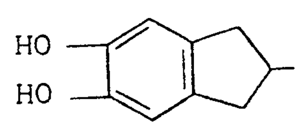

.

Preferred anorectic agents of formula (I) are the D-amino acid oxidase substrates of the formula:

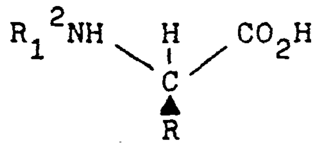

(Ib)

wherein the R moiety is as defined for formula (I) and $R_1^2$ is the des-amino residues of such compounds as:

α-methyl-4-(trifluoromethyl)phenethylamine,
α-methyl-3-(trifluoromethyl)phenethylamine,
β,β-difluoroamphetamine,
2-amino-1-phenylpropanol (norisoephedrin),
2-(1-aminopropyl)-2-indanol (indanazoline),
2-chloro-α,α-dimethylphenethylamine (chlortermine),
4-chloro-α,α-dimethylphenethylamine (chlorphentermine),
α,α-dimethylphenethylamine (phentermine),
2-amino-1-(2'-methylphenyl)propane (ortetamine),
2-phenyl-3-methyl-3-butylamine (pentorex).

Preferred centrally acting compounds of formula (I) useful for the treatment of hypertension are the D-amino acid oxidase substrates wherein the $R_1$ therapeutic agents are the des-amino residues of α-methyl dopamine and α-methyl norepinephrine.

The listing of therapeutic agents which have been found to be useful in the treatment of mental depression represents a very large class of compounds. These compounds, generally referred to as antidepressants, can be characterized as (1) MAO inhibitors, both irreversible and competitive inhibitors, (2) "tricyclics", which are believed to interfere with the up-take of norepinephrine or serotonin by the nerve cell and (3) a miscellaneous group of compounds which do not fall into either of the foregoing general classes. The preferred antidepressant agents of formula (I) are the D-amino acid oxidase substrates wherein the $R_1$ moiety represents the des-amino residues of compounds such as: 4-dimethylamino-α-2-dimethylphenethylamine, tranylcypromine, etryptamine, compounds having the structural formulae:

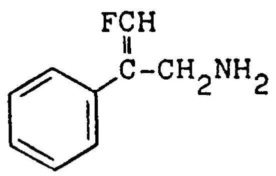

or

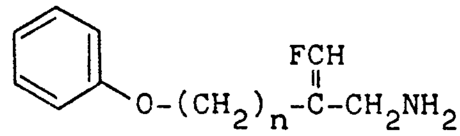

and their steric isomers, wherein each of the phenyl moieties may bear 1, 2 or 3 substituents selected from the group: lower alkyl, alkoxy, hydroxy, halogen or $CF_3$; and the $(CH_2)_n$ can be an alkylene moiety having 1 to 11 carbon atoms.

It is believed that any of the tricyclic antidepressants presently in use today, were they to contain a $-NH_2$ moiety (rather than in their existing secondary or tertiary amine forms), would be suitable as $R_1$ des-amino residues and would be useful substrates for D-amino acid oxidase. Thus, des-amino compounds of antidepressant tricyclics having the formulae:

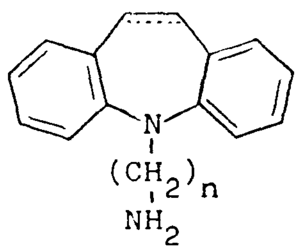

(a)

or

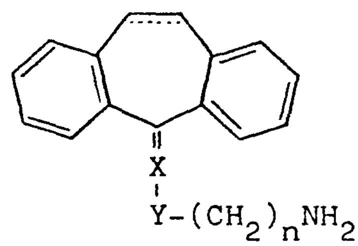

(b)

wherein the dotted line is a facultative double bond, the group $(CH_2)_n$ of formula (a) is a straight or branched lower alkyl group containing at least 3 carbon atoms to separate the two nitrogen atoms, and for formula (b) X is nitrogen, Y is oxygen and n is 2; or X is carbon, Y is $CH_2$ and n is one.

In the field of Angiotensin Converting Enzyme (ACE) inhibitors which are useful as antihypertensive agents, it is preferred to use the des-amino residues of compounds having the formula:

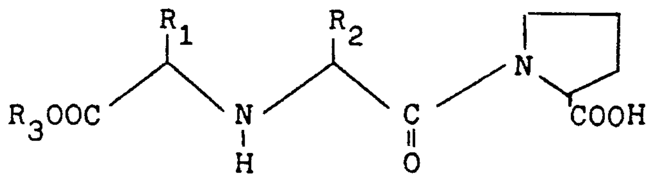

wherein

$R_1$ is $(CH_2)_2C_6H_5$ or $(CH_2)_2$-CH 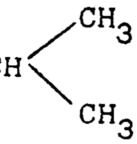 ,

$R_2$ is $(CH_2)_4NH_2$ and

$R_3$ is H or lower alkyl ($C_{1-4}$).

In the area of growth promoting agents, it is preferred to use the des-amino residues of peptides such as, for example, histidine-D-tryptophane-alanine-tryptophane-D-phenylalanine-lysine.

Similarly, in the area of analgesia $R_1$ may represent the des-amino residues of a peptide, as for example, tyrosine-glycine-glycine-phenylalanine-leucine (leucine-enkephalin) or tyrosine-glycine-glycine-phenylalanine-methionine (methionine-enkephalin).

In the area of diuretics the symbol $R_1$ of formula (I) may represent the des-amino residues of various diuretics as long as the diuretic agent does not contain a primary amine adjacent to an aryl or aromatic heterocyclic ring, or an α-carboxylic acid or ester adjacent to the primary amine. Thus, derivatives of dopamine and related congeners, in addition to their other properties, exert a diuretic effect upon renal vasodilation and sodium excretion. Accordingly, their des-amino residues form useful substrates in the practice of this invention.

Representative of the preferred anti-migraine compounds are those D-amino acid oxidase substrates wherein the symbol $R_1$ of formula (I) is a des-amino residue of

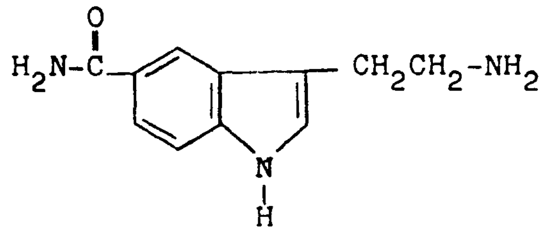

Representative of the antibiotic compounds of formula (I) useful as therapeutic agents are the D-amino acid oxidase substrates in which the $R_1$ moiety represents the des-amino residues of aminoglycosides, such as gentamicin, kanamycin, amikacin, paramomycin, tobramycin and the antimalarial, primaquine.

In general, the chemical compounds of this invention may be prepared by the application of analogous chemical reactions known to the art. Such processes may conveniently be characterized as (1) the N-alkylation of D-amino acid esters, (2) the N-alkylation of N-protected D-amino acid esters, (3) the N-alkylation of amine derivatives, (4) the reductive alkylation of the D-amino acid and its esters and (5) the reductive alkylation of the primary amine. Quite naturally these processes are not the only processes available but they represent the preferred synthetic methods for the preparation of compounds of formula (I). The choice of any one of these processes depends upon the usual parameters generally known to those of ordinary skill in the art as well as the ready availability of the required reactants.

In its general aspects the N-alkylation of D-amino acid esters may be depicted as follows:

<u>Reaction Scheme A</u>

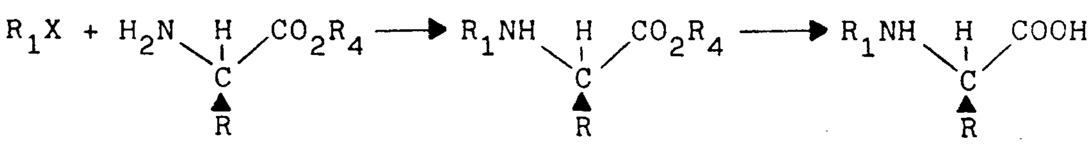

(II) (III) (IV) (I)

In the above reaction, R and $R_1$ are as defined in formula (I); the symbol X is any suitable leaving group, such as halogen, e.g., Cl, Br or I, tosylate, mesylate and the like; and the symbol $R_4$ is hydrogen or a staight or branched chain lower alkyl group ($C_{1-6}$).

In conducting the foregoing reaction, equivalent quantities of reactants, using the D-form of the amino acid (II), are reacted in an organic solvent such as dimethoxyethane, dimethylformamide, dimethylsulfoxide, tetrahydrofuran and the like. The reaction is conducted in the presence of one equivalent of a base such as triethylamine or its functional equivalents at temperatures ranging from 20° to 180°C for a period of from 1 to 48 hours. Upon completion of the reaction, the N-alkylated ester is hydrolyzed to the corresponding acid using standard acidic or basic conditions, e.g., LiOH in a water/alcohol mixture or refluxing aqueous HBr or HCl.

The N-alkylation of N-protected D-amino acid esters can be depicted as follows:

<u>Reaction Scheme B</u>

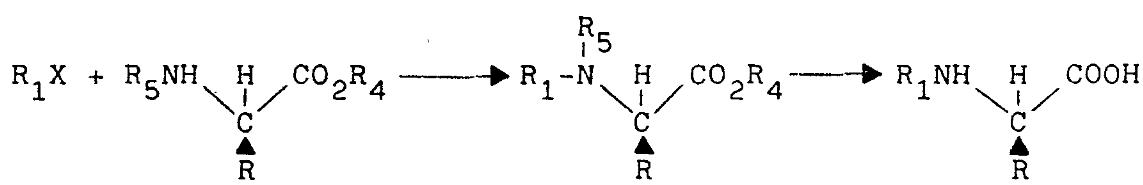

(II) (V) (VI) (I)

In the above reaction the symbols R, $R_1$, $R_4$ and X are as previously defined, and the symbol $R_5$ represents an amine protecting group such as $C_6H_5\overset{O}{\overset{\|}{C}}-$, $C_6H_5CH_2O\overset{O}{\overset{\|}{C}}-$ or $R_6O\overset{O}{\overset{\|}{C}}-$, where $R_6$ is a lower alkyl group with the t-butyl group a particularly preferred group.

In conducting the foregoing reaction, the N-protected amino acid (V) is treated with one equivalent of a strong base in an aprotic solvent, such as tetrahydrofuran, dimethylformamide, and the like. The base should be capable of abstracting the hydrogen from the N-protected amine function and can, for example, be sodium hydride, potassium hydride, lithium diisopropyl amide, and the like. One or two equivalents of the reactant (II) above are added to the anion, and the resulting mixture is stirred for a period of from 1 to 2 hours at a temperature ranging from about -7° to 100°C. The N-alkyl derivative (VI) obtained in this fashion can be purified via silica gel chromatography or utilizing crystallization techniques readily available to those skilled in the art. The desired compounds (I) are obtained via cleavage of the ester and the N-protecting groups utilizing standard conditions known to those skilled in the art.

The N-alkylation of amine derivatives can be depicted in the following schematic representation:

Reaction Scheme C

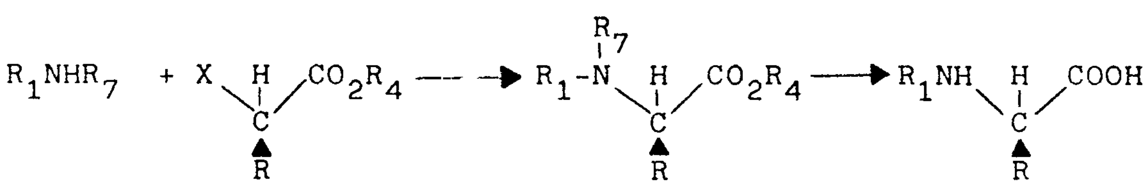

(VII) (VIII) (IX) (I)

In the above reaction the symbols R, $R_1$, $R_4$ and X are as previously defined, and $R_7$ represents hydrogen or an N-protecting group, such as $C_6H_5\overset{O}{\overset{\|}{C}}-$ , $R_6O\overset{O}{\overset{\|}{C}}-$ , wherein $R_6$ is as previously defined, $C_6H_5O\overset{O}{\overset{\|}{C}}-$ or $R_8SO_2-$ , wherein $R_8$ represents aryl.

When the symbol $R_2$ is hydrogen, the foregoing reaction can be effected in an inert organic solvent, such as dimethoxyethane, dimethylformamide, dimethylsulfoxide, tetrahydrofuran, etc. Equivalent quantities of the appropriate acid derivatives (VIII) are reacted with the N-protected amine (VII) in the presence of one equivalent of a base, such as triethylamine, for a period of from 1 to 24 hours at temperatures ranging from about 0° to 100°C.

The resulting N-alkylated-D-amino acid ester derivatives can be purified utilizing silica gel chromatography or crystallization techniques known to those skilled in the art. The desired D-amino acid substrates (I) can be readily obtained upon cleavage of the corresponding esters (IX) utilizing procedures previously described. When the symbol $R_7$ represents an N-protecting group, the process of Reaction Scheme B is applied.

The reductive alkylation of D-amino acid and its esters can be depicted in the following schematic representation:

Reaction Scheme D

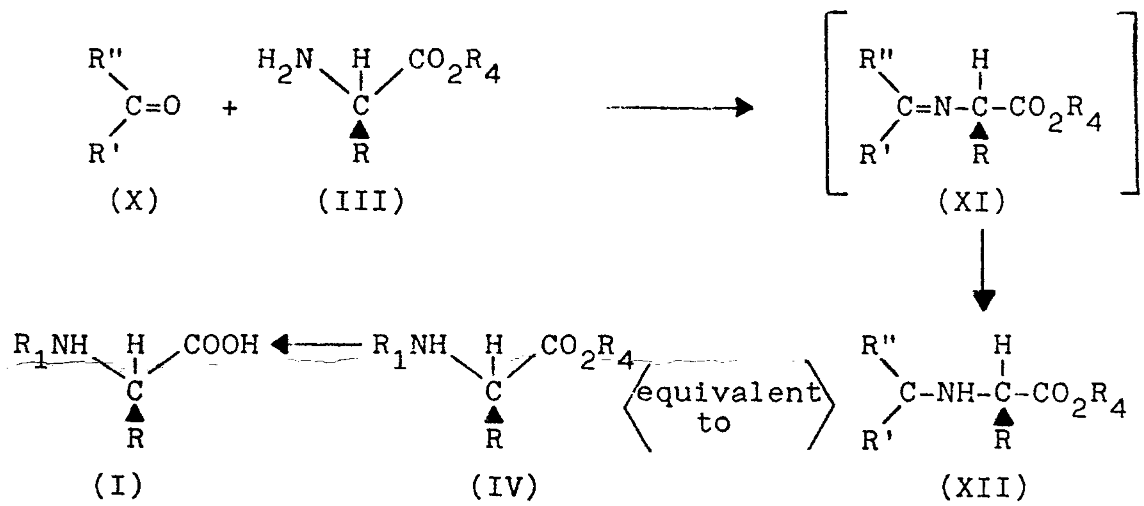


(X) (III) (XI)

(I) (IV) (XII)

In the above reaction the symbols R, $R_1$, $R_4$ are as previously defined and the structural representation

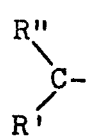

represents an equivalent moiety for the symbol $R_1$. Thus, the ketone (X) above

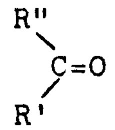

represents the corresponding keto-derivative of the des-amino bearing therapeutic agent.

In effecting the reductive alkylation, conditions well known to those skilled in the art are employed, see R.F. Borsch <u>et al.</u>, JACS <u>93</u>, 2897 (1971). Thus, the carbonyl derivative (X) and the D-amino acid or its ester (III) are reacted in the presence of a reducing agent, such as hydrogen, and a suitable catalyst. Suitable catalysts employed include Pd/C, $PtO_2$, $BH_4Na$, $BH_3NaCN$ and the like. In those instances in which the borohydrides are employed, it is important to control the pH of the reaction mixture to a pH of about 6 to 8. The reductive alkylation proceeds as a single step. Thus, the hypothetical imine intermediate (XI) is never isolated, but proceeds directly to the corresponding amine (XII). De-esterification of (XII), or its equivalent structure (IV), is conducted as previously described.

The reductive alkylation of a primary amine can be depicted as follows:

<u>Reaction Scheme E</u>

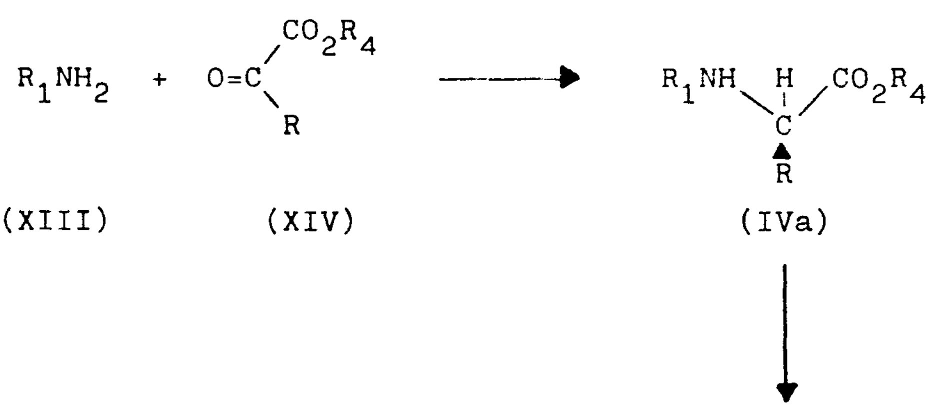

(XIII) (XIV) (IVa)

(IVa)

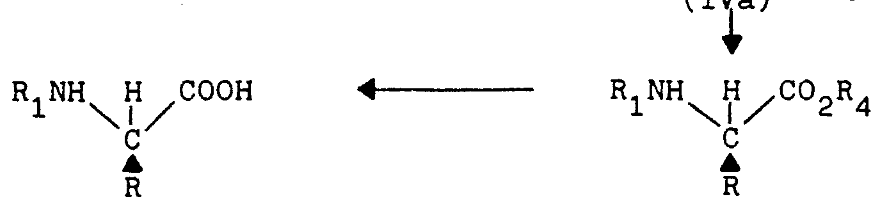

(I) (IV)

In the above reaction the symbols R, $R_1$ and $R_4$ are as previously defined. In general, the reaction conditions are the same as described for Reaction Scheme D. However, the amino acid derivatives (IVa) are obtained as racemic mixtures. The amino acid derivatives (IVa) can be resolved to the desired D-enantiomers of the corresponding D-amino acid derivatives (IV) using standard resolution techniques well known to those skilled in the art. De-esterification of (IV) then proceeds in the manner previously described.

Alternatively the racemic amino acid derivatives (IVa) can be de-esterified to yield the racemic amino acid oxidase substrates of (I), which are then resolved to provide the desired D-amino acid oxidase substrates (I) of the present invention.

The following specific examples illustrate the foregoing generally described reactions. Utilizing the appropriate reaction, any of the compounds of formula (I) may readily be obtained when the appropriate derivatives of compounds $R_1NH_2$ and 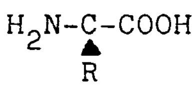 are utilized.

## Example 1

## N-(3,4-Dihydroxyphenethyl)glycine

A solution of the N-*tert*-butoxycarbonyl derivative (1.12 g), available from β-3,4-dimethoxyphenethylamine and di-*tert*-butyldicarbonate, in THF (15 ml) is added slowly to cold (*ca*. -70°C) LDA (prepared from 0.40 g of diisopropylamine and 2.7 ml of 1.5 M n-butyllithium) in THF (15 ml). After 30 minutes, a solution of *tert*-butyl bromoacetate (0.72 g) in THF (5 ml) is added, the cooling bath is removed and the reaction stirred overnight. The solvent is evaporated and the

product is subsequently isolated by ether extraction as a pale orange oil (1.10 g). Chromatography (silica; 40% ether in light petroleum) affords essentially pure protected amino acid (0.66 g) as a colorless oil.

A portion of this product (0.50 g) in 47% aqueous HBr (20 ml) is refluxed for 5 hours, then evaporated to dryness. The solid residue (0.29 g) is dissolved in EtOH (5 ml) and treated with an excess of propylene oxide (0.18 g); theresulting precipitate is collected and dried to yield N-(3,4-dihydroxyphenethyl)-glycine as a greyish powder (0.20 g): m.p. 245-256° (dec.).

## Example 2

## N-[(E)-3-fluoro-2-(3-trifluoromethylphenyl)-2-propenyl]glycine

The N-*tert*-butoxycarbonyl derivative 2 (0.96 g), prepared from (E)-2-(3-trifluoromethylphenyl)-3-fluoro-allylamine and di-*tert*-butyl dicarbonate in the usual way, is dissolved in dimethylformamide (DMF, 20 ml) and cooled to *ca.* -10°C. A dispersion of sodium hydride in mineral oil (0.14 g; *ca.* 55% NaH) is added and, after stirring for 20 minutes, a solution of *tert*-butyl bromoacetate (0.55 g) in DMF (1 ml) is added dropwise. Stirring is continued for 1 hour at -10°C then water is added and the product is isolated by ether extraction. The crude product is purified by silica chromatography (using 20% ether in light petroleum eluant) whereupon the protected amino acid is obtained as a colorless oil (0.63 g; 48% yield).

This substance is dissolved in trifluoroacetic acid (20 ml) at room temperature, then evaporated to dryness after 1 hour. The residue is taken up in $CH_3OH$ (5 ml) and treated with propylene oxide (1 ml) at -20°C overnight. The resulting precipitate is collected and dried affording the desired glycine derivative (0.12 g; 30% yield) as colorless needles; m.p. 199-201°C (dec.).

### Example 3

### N-[6,7-Dihydroxy-2-tetralyl]glycine

A mixture of 6,7-dimethoxy-2-tetralone (2.06 g) and tert-butyl glycinate (1.3 g) in anhydrous tetrahydrofuran (20 ml) was stirred at room temperature for 4 hours with n° 4A powdered molecular sieves. Sodium cyanoborohydride (0.6 g) was added in two portions of 0.3 g at 6 hour intervals. The reaction mixture was then filtered and the filtrate was concentrated *in vacuo*. The residue was taken up in diethyl ether (50 ml). The organic phase was washed with water, brine, dried over $MgSO_4$ and concentrated *in vacuo*. The residue was subjected to chromatography on silica gel to yield N-tert-butyloxycarbonylmethyl-6,7-dimethoxy-2-aminotetraline, which was refluxed in 47% aqueous hydrobromic acid for 5 hours. The solid residue obtained upon concentration, *in vacuo*, was dissolved in ethanol and treated with an excess of propylene oxide to yield the title compound.

### Example 4

### N-[3-3-Trifluoromethylphenyl)-2-propyl]alanine

A mixture of a α-methyl-3-trifluoromethylbenzene ethanamine (1.9 g), (S)-tert-butyl-2-bromopropionate (2.09 g) and triethylamine (1.4 ml) in dimethylformamide (20 ml) was stirred at room temperature for 16 hours. The solution was then concentrated *in vacuo* and the residue was extracted with diethylether (50 ml). The organic phase was washed with water, brine, dried over $MgSO_4$ and concentrated *in vacuo*. The residue was chromatographed on silica gel to give (R)-tert-butyl N-[3-(3-trifluoromethylphenyl)-2-propyl]alanate which is hydrolyzed to the desired product.

The determination of whether or not a particular compound embraced within the generic concept of this invention would be a suitable substrate for D-amino acid oxidase may be made on the basis of a simple *in vitro* test

and confirmed with standard laboratory in vivo techniques. For the in vitro testing, using commercial hog kidney D-amino acid oxidase, the compounds are incubated under optimum conditions of the enzymatic reaction, and either formation of $H_2O_2$ or that of the expected primary amine is quantitatively determined as a function of substrate concentration, incubation time, etc.

In general, the dose and means of administration of the compounds of this invention is determined by standard evaluation techniques known for the particular class of compounds to which the amino-bearing therapeutic agent belongs.

CLAIMS

1. A D-amino acid oxidase substrate of the structural formula

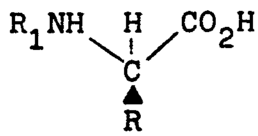

and the pharmaceutically acceptable salts thereof, wherein R is H, $-CH_3$, $CH_3CH_2-$, $CH_3CH_2CH_2-$, $CH_3(CH_2)_2CH_2-$, $(CH_3)_2CH-$, $CH_3CH_2\overset{CH_3}{\overset{|}{C}}H-$, $-CH_2OH$, $CH_3CH_2SCH_2CH_2-$, $CH_3SCH_2-$, $CH_3SCH_2CH_2-$, $C_6H_5CH_2-$, $4\text{-}OH\text{-}C_6H_4CH_2-$, $3,4\text{-diOH-}C_6H_3CH_2-$ and

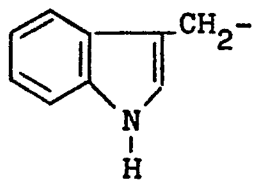

and $R_1$ is the des-amino residue of a $-NH_2$ bearing therapeutic agent.

2. A D-amino acid oxidase substrate of Claim 1 wherein R is H, $-CH_3$ or $(CH_3)_2CH-$.

3. A D-amino acid oxidase substrate of Claim 1 suitable for use as an antihypertensive agent, wherein $R_1$ has the structure

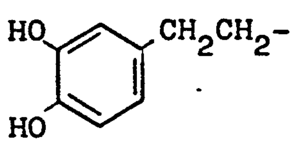

,

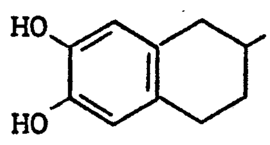

,

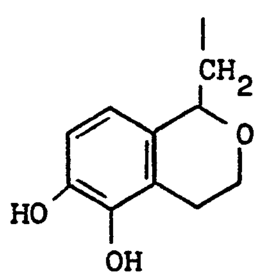

,

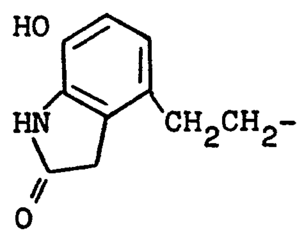

,



C-32,516A

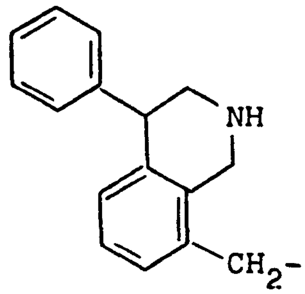 , 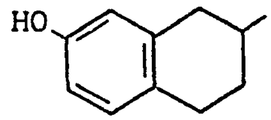 ,

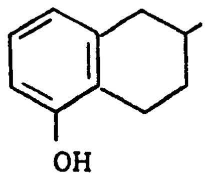 , 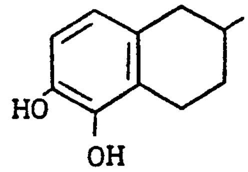 ,

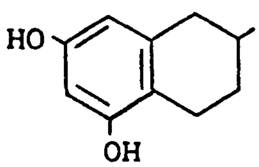 , and 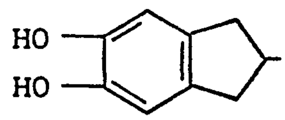 .

4. A D-amino acid oxidase substrate of Claim 1 suitable for use as an antidepressant, wherein $R_1$ represents the des-amino residue of 4-dimethylamino-α-2-dimethylphenethylamine, tranylcypromine, etryptamine, or compounds having the formula

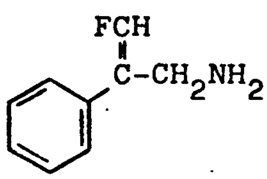 or 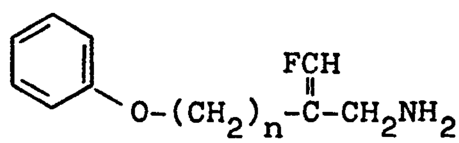

wherein each of the phenyl moities may bear 1, 2 or 3 substituents selected from the group of lower alkyl, alkoxy, hydroxy, halogen or $CF_3$; and the $(CH_2)_n$ is an alkylene moiety having from 1 to 11 carbon atoms.

5. A D-amino acid oxidase substrate of Claim 1 suitable for use as an antihypertensive agent wherein $R_1$ is the des-amino residue of compounds of the formula

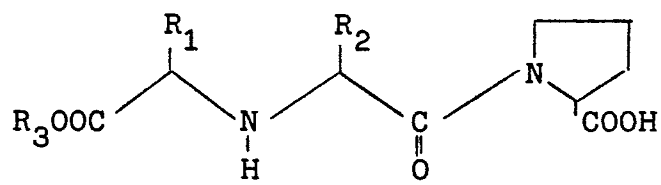

wherein

$R_1$ is $(CH_2)_2C_6H_5$ or 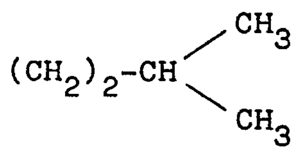 ,

$R_2$ is $(CH_2)_4NH_2$ and

$R_3$ is H or lower alkyl $(C_{1-4})$.

6. A compound according to Claim 1 which is N-(3,4-dihydroxyphenethyl)glycine or the pharmaceutically acceptable salt thereof.

7. A process for the preparation of D-amino acid oxidase substrates having the formula

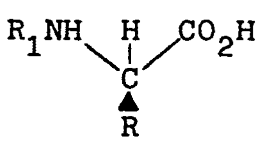

wherein R is H, $-CH_3$, $CH_3CH_2-$, $CH_3CH_2CH_2-$, $CH_3(CH_2)_2CH_2-$,

$(CH_3)_2CH-$, 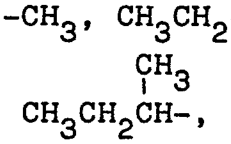, $-CH_2OH$, $CH_3CH_2SCH_2CH_2-$, $CH_3SCH_2-$, $CH_3SCH_2CH_2-$, $C_6H_5CH_2-$, 4-OH-$C_6H_4CH_2-$, 3,4-diOH-$C_6H_3CH_2-$ and

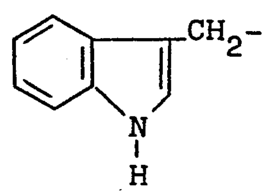

, and



C-32,516A

$R_1$ is the des-amino residue of an amino bearing therapeutic agent; which comprises the N-alkylation of a derivative of said therapeutic agent having the formula

$$R_1NHR_7$$

wherein $R_7$ represents hydrogen, $C_6H_5\overset{O}{\overset{\|}{C}}-$, $R_6O\overset{O}{\overset{\|}{C}}-$, $C_6H_5O\overset{O}{\overset{\|}{C}}-$ or $R_8SO_2$ in which $R_6$ represents lower alkyl having 1-4 carbon atoms and $R_8$ represents aryl; with a D-acid derivative having the formula

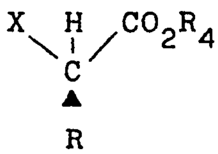

wherein X is halogen, tosylate or mesylate, and
$R_4$ is hydrogen or a straight or branched chain lower alkyl group having 1-6 carbon atoms in the presence of a base for a period of from 1 to 24 hours and at temperature ranging from 0 to 100°C; with the subsequent removal of the ester and amine protecting groups to form the D-amino acid oxidase substrate.

8. A process according to Claim 7 wherein R is H, $-CH_3$ or $(CH_3)_2CH-$.

9. A process according to Claim 7 wherein $R_1$ has the structure

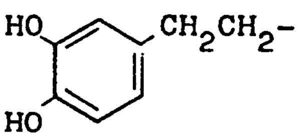

,

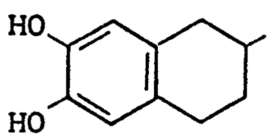

,

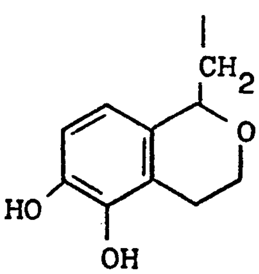

,

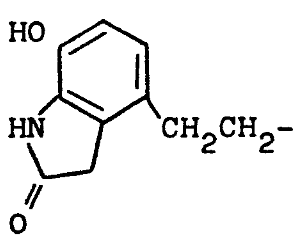

,

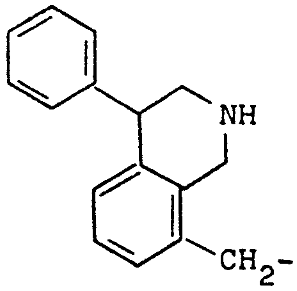

,

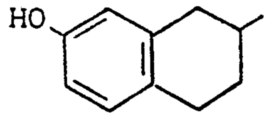

,

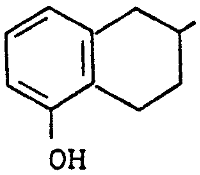

,

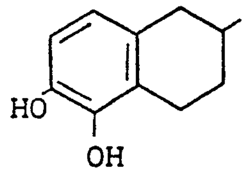

,

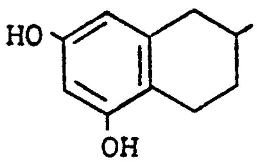

, and

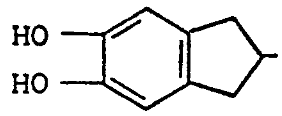

.

10. A process according to Claim 7 wherein $R_1$ is the desamino residue of compounds of the formula

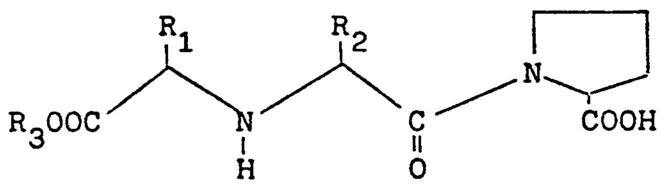

wherein

$R_1$ is $(CH_2)_2C_6H_5$ or 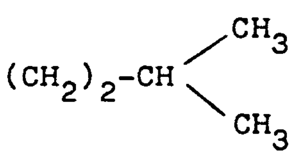 ,

$R_2$ is $(CH_2)_4NH_2$ and

$R_3$ is H or lower alkyl $(C_{1-4})$.

11. A compound of claim 1 for use as a medicine.



C-32,516A

12. A pharmaceutical composition comprising a compound of Claim 1 in admixture or otherwise associated with a pharmaceutically acceptable diluent or carrier.